(19) Europäisches Patentamt
European Patent Office
Office européen des brevets

(11) Veröffentlichungsnummer: **0 199 168**
**A1**

# (12) EUROPÄISCHE PATENTANMELDUNG

(21) Anmeldenummer: **86104719.9**

(22) Anmeldetag: **07.04.86**

(51) Int. Cl.⁴: **C07D 249/08**, C07D 403/06, C07D 405/06, A01N 43/653

(30) Priorität: **17.04.85 DE 3513714**

(43) Veröffentlichungstag der Anmeldung: **29.10.86 Patentblatt 86/44**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Kraatz, Udo, Dr.**
**Andreasstrasse 22a**
**D-5090 Leverkusen 1(DE)**
Erfinder: **Büchel, Karl Heinz, Prof. Dr.**
**Dabringhausener Strasse 42**
**D-5093 Burscheid(DE)**
Erfinder: **Jäger, Gerhard, Dr.**
**Paul-Klee-Strasse 28j**
**D-5090 Leverkusen(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen(DE)**

## (54) Bisazolylethercarbinole.

(57) Die Erfindung betrifft neue Bisazolylethercarbinole, ein Verfahren zu ihrer Herstellung und ihre Verwendung als Fungizide.

Die Verbindungen der Formel

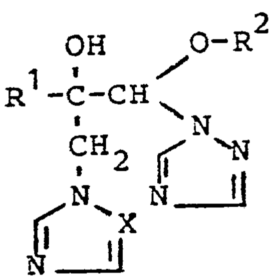

(I)

EP 0 199 168 A1

in welcher

$R^1$, $R^2$ uns X die in der Beschreibung angegebenen Bedeutungen besitzen, werden erhalten,

wenn man die entsprechenden Azolyl-phenoxymethyl-oxirane mit einem Azol umsetzt.

Die Verbindungen besitzen eine gute Wirksamkeit gegen phytopathogene Pilze.

# Bisazolylethercarbinole

Die vorliegende Erfindung betrifft neue Bisazolylethercarbinole, ein Verfahren zu ihrer Herstellung und ihre Verwendung als Fungizide.

Es ist bereits bekannt geworden, daß allgemein 1-Phenoxy-1-(1,2,4-triazol-1-yl)-3,3-dimethyl-butan-2-on-Derivate eine sehr gute fungizide Wirksamkeit besitzen (vgl. z.B. DE-A 2 201 063 bzw. die entsprechende US-PS 3 912 752). In einzelnen Anwendungsbereichen ist jedoch bei niedrigen Aufwandmengen die Wirksamkeit nicht immer voll befriedigend.

Es wurden neue Bisazolylethercarbinole der allgemeinen Formel (I)

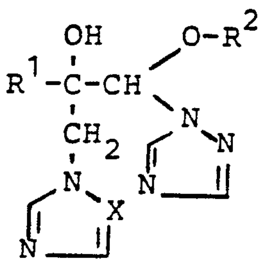

(I)

in welcher

$R^1$ für gegebenenfalls substituiertes Alkyl, Cycloalkyl, für gegebenenfalls substituiertes Aryl und für einen gegebenenfalls substituierten 5-oder 6-gliedrigen Heterocyclus steht,

$R^2$ für gegebenenfalls substituiertes Aryl und für einen gegebenenfalls substituierten 5-oder 6-gliedrigen Heterocyclus steht, und

X für Stickstoff oder die CH-Gruppe steht,

und deren Säureadditionssalze und Metallsalzkomplexe gefunden.

Man erhält die Bisazolylethercarbinole der Formel (I) sowie deren Säureadditions-Salze und Metallsalz-Komplexe,

wenn man Azolyl-phenoxy-methyl-oxirane der allgemeinen Formel (II)

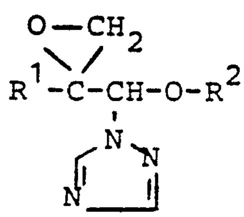

(II)

in welcher

$R^1$ und $R^2$ die oben angegebene Bedeutung besitzen,

mit einem Azol der Formel (III)

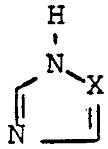

(III)

in welcher

X die oben angegebene Bedeutung besitzt,

gegebenenfalls in Gegenwart eines Lösungsmittels umsetzt, und gegebenenfalls noch an die erhaltenen Verbindungen der Formel (I) anschließend eine Säure oder ein Metallsalz addiert.

Die neuen Bisazolylethercarbinole der allgemeinen Formel (I) sowie deren Säureadditions-Salze und Metallsalz-Komplexe weisen starke fungizide Eigenschaften auf. Dabei zeigen die erfindungsgemäßen Stoffe überraschenderweise eine erheblich höhere fungizide Wirkung als die aus dem Stand der Technik bekannten 1-Phenoxy-1-(1,2,4-triazol-1-yl)-3,3-dimethyl-butan-2-on-Derivate.

Von den erfindungsgemäßen Bisazolylethercarbinolen der allgemeinen Formel (I) sind bevorzugt diejenigen, in denen

$R^1$ für Alkyl mit 1 bis 6 Kohlenstoffatomen steht, welches substituiert sein kann durch Halogen, Methoxy, Methylthio und Cyano, für Cycloalkyl mit 5 bis 6 Kohlenstoffatomen und weiterhin für Phenyl steht, wobei letzteres gegebenenfalls substituiert sein kann durch Halogen, Alkyl mit 1 bis 4 C-Atomen, Methoxy, Methylthio, Trifluormethyl, Trifluormethoxy und Nitro, und schließlich für einen 5- oder 6-gliedrigen Heterocyclus mit 1 oder 2 Stickstoffatomen und/oder einem Sauerstoff-oder Schwefelatom steht, der substituiert sein kann durch Halogen, Methyl, Methoxy und Methylthio,

$R^2$ für Phenyl steht, welches gegebenenfalls durch die bei $R^1$ genannten Phenylsubstituenten substituiert sein kann, und schließlich für einen 5-oder 6-gliedrigen Heterocyclus mit 1 oder 2 Stickstoffatomen und/oder einem Sauerstoff-oder Schwefelatom steht, der substituiert sein kann durch Halogen, Methyl, Methoxy und Methylthio, und

X für Stickstoff oder die CH-Gruppe steht.

Besonders bevorzugt sind diejenigen Bisazolylethercarbinole, in denen

$R^1$ für Alkyl mit 1 bis 4 Kohlenstoffatomen steht, welches gegebenenfalls durch Halogen oder Methoxy substituiert sein kann, weiterhin für Phenyl steht, welches gegebenenfalls ein-bis dreifach durch Fluor, Chlor, Brom, Methyl, Methoxy und/oder Trifluormethyl substituiert sein kann, und - schließlich für den Pyridyl-oder Thienyl-Rest steht,

$R^2$ für Phenyl steht, welches gegebenenfalls ein-bis dreifach durch die bei $R^1$ genannten Phenylsubstituenten substituiert sein kann, und schießlich für den Pyridylrest steht, und

X für Stickstoff oder die CH-Gruppe steht.

Bevorzugte erfindungsgemäße Verbindungen sind auch Additionsprodukte aus Säuren und denjenigen Bisazolylethercarbinolen der Formel (I), in denen die Substituenten $R^1$, $R^2$ und X die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt-und der I. und II. sowie IV. bis VIII. Nebengruppe des Periodensystems der Elemente und denjenigen Verbindungen der Formel (I), in denen die Substituenten $R^1$, $R^2$ und X die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprdoukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man zur Herstellung der erfindungsgemäßen Bisazolylethercarbinole der Formel (I) beispielsweise 2-[(4-Chlorphenoxy)-(1,2,4-triazol-1-yl)-methyl]-2-(tert.-butyl)-oxiran und 1,2,4-Triazol als Ausgangsstoffe, so kann der Reaktionsablauf durch das folgende Formelschema wiedergegeben werden:

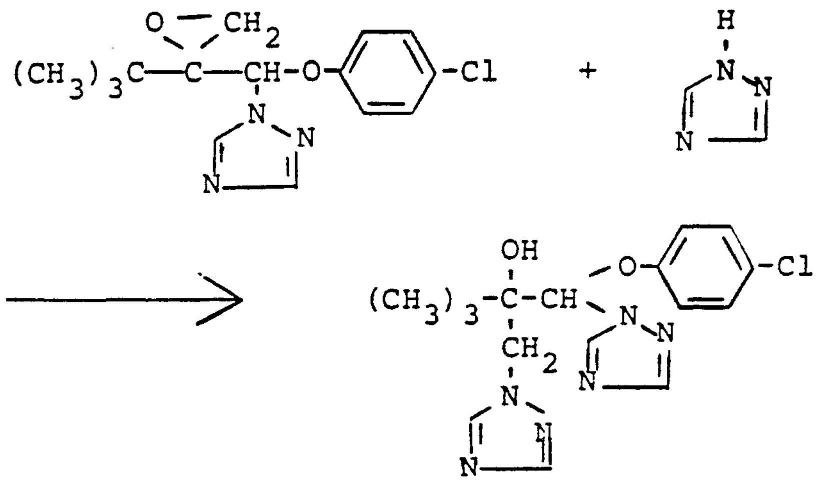

Die zur Herstellung der erfindungsgemäßen Verbindungen der Formel (I) als Ausgangsstoffe benötigten Azolyl-phenoxy-methyl-oxirane sind durch die Formel (II) allgemein bezeichnet.

In dieser Formel haben $R^1$ und $R^2$ vorzugsweise die weiter oben angegebenen Bedeutungen.

Die Verbindungen der Formel (II) sind noch nicht bekannt; sie stellen interessante neue Zwischenprodukte dar. Die Verbindungen besitzen außerdem selbst eine fungizide Wirksamkeit.

Die Azolyl-phenoxy-methyl-oxirane der Formel (II) werden in allgemein bekannter Weise erhalten, wenn man die entsprechenden Azolyl-phenoxy-keto-Verbindungen der Formel (IV)

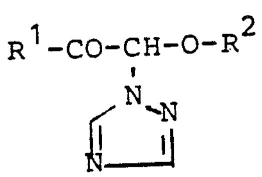

(IV)

in welcher

$R^1$ und $R^2$ die weiter oben angegebenen Bedeutungen besitzen,

mit Trimethylsulfoxoniumjodid (oder mit Trimethylsulfoniumjodid) in einem polaren Lösungsmittel in Gegenwart einer starken Base zur Reaktion bringt (Epoxidierungs-Reaktion nach Corey, vgl. hierzu J. Am. Chem. Soc. 87 , 1363-64 (1965)).

Die als Vorprodukte zur Herstellung der Verbindungen der allgemeinen Formel (II) benötigten Azolyl-phenoxy-keto-Verbindungen der Formel (IV) sind schon seit längerer Zeit allgemein bekannt - (vgl. z.B. R. Wegler, "Chemie der Pflanzenschutz- und Schädlingsbekämpfungsmittel", Springer-Verlag Berlin/Heidelberg/New York (1977), Band 4, Seite 208).

Die zur Herstellung der erfindungsgemäßen Verbindundgen der Formel (I) als Ausgangsstoffe weiterhin benötigten Azole sind durch die Formel - (III) wiedergegeben. Die unter die Formel (III) fallenden beiden Verbindungen Imidazol und 1,2,4-Triazol sind schon seit langer Zeit allgemein bekannte und laboratoriumsübliche Stoffe.

Als Verdünnungsmittel kommen zur Herstellung der erfindungsgemäßen Verbindungen der Formel (I) organische Lösungsmittel in Frage. Hierzu gehören Kohlenwasserstoffe, wie Benzin, Toluol, Ether, wie Diethylether, Dioxan und insbesondere polare Stoffe wie Alkohole, so z.B. Methanol, Ethanol und Butanol, oder auch Acetonitril.

Zur Herstellung der Zwischenprodukte der Formel (II) kommen ebenfalls organische Lösungsmittel als Verdünnungsmittel in Frage. Hierzu gehören Kohlenwasserstoffe, wie Benzin, Toluol, Ether, wie Diethylether, Dioxan und insbesondere polare Stoffe wie z.B. Dimethylsulfoxid, tert.-Butanol, Acetonitril.

Als Hilfsstoffe werden zur Herstellung der erfindungsgemäßen Verbindungen der Formel (I) stark alkalische Stoffe verwendet. Hierzu eignen sich vorzugsweise Alkalimetallalkoholate, wie z.B. Natrium-methylat oder Natrium-butylat.

Zur Herstellung der Zwischenprodukte der Formel (II) werden als Hifsstoffe Säurebindemittel benötigt. Hierzu eignen sich vorzugsweise starke Basen, wie z.B. Kalium-tert.-butylat, Natrium-methylat, Natrium-hydrid.

Die Reaktionstemperaturen können bei der Herstellung der erfindungsgemäßen Verbindungen der Formel (I) in einem größeren Bereich variiert werden. Im allgemeinen arbeitet man bei Temperaturen zwischen etwa 50 und 150°C, vorzugsweise zwischen 80 und 120°C.

Zur Herstellung der Zwischenprodukte der Formel (II) können die Reaktionstemperaturen ebenfalls in einem größeren Bereich variiert werden. Im allgemeinen arbeitet man zwischen 0 und 130°C, vorzugsweise zwischen 20 und 90°C.

Bei der Durchführung des Verfahrens zur Herstellung der erfindungsgemäßen Verbindungen der Formel (I) werden auf 1 Mol an Zwischenprodukt der Formel (II) etwa 1 bis 2 Mol Azol der Formel - (III) und 0,1 bis 0,7 Mol Natriumalkoholat eingesetzt. Die Aufarbeitung erfolgt in üblicher Weise durch Einengen des Reaktionsgemisches und Waschen mit Wasser. Nach anschließendem Trocknen kann die Reinigung chromatographisch erfolgen.

Zur Herstellung der Zwischenprodukte der Formel (II) werden auf σ Mol an Azoyl-phenoxy-keto-Verbindung der Formel (IV) etwa 1 bis 1,5 Mol an Trimethylsulfoxoniumjodid und 1 bis 1,5 Mol an Alkalihydrid eingesetzt. Die Aufarbeitung erfolgt in üblicher Weise durch Eintragen des Reaktionsgemisches in Wasser, Aufnehmen in Lösungsmittel, Trocknen und Einengen.

Die nach dem erfindungsgemäßen Verfahren erhältlichen Verbindungen der Formel (I) können in Säureadditions-Salze bzw. Metallsalz-Komplexe überführt werden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsure, erhalten werden und in bekannter Weise, z.B. durch Abfiltrieren, isoliert und gegebenenfalls durch Waschen mit einem inerten organischen Lösungsmittel gereinigt werden.

Die Metallsalz-Komplexe von Verbindungen der Formel (I) können ebenfalls nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Um¸ kristallisation reinigen.

Von den neuen Zwischenprodukten der Formel (II) können gegebenenfalls auch die Säureadditions-Salze und Metallsalz-Komplexe hergestellt werden. Die Darstellung erfolgt in der oben angegeben allgemein bekannten Art und Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt sur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryxae;

Pseudomonas-Arten, wie beispielsweise Pseudomonas syringae pv. lachrymans;

Erwina-Arten, wie beispielsweise Erwina amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea;

(Konidienform: Drechslera, Syn: Helminthosporium);

Cochliobolus-Arten, wie beispielsweise Cochliobolus sativus;

(Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe der allgemeinen Formel (I) mit besonders gutem Erfolg zur Bekämpfung von Krankheiten in Getreide und Reiskulturen Verwendung finden; so z.B. gegen Mehltau, gegen Rost, sowie gegen durch Fusarium-Arten und durch Pyrenophora teres und durch Pyricularia oryzae hervorgerufene Pflanzenkrankheiten.

Wie schon angegeben, besitzen nicht nur die erfindungsgemäßen Bisazolylethercarbinole der allgemeinen Formel (I), sondern auch die als Zwischenprodukte fungierenden Azolyl-phenoxymethyl-oxirane der allgemeinen Formel (I) eine beachtliche fungizide Wirkung. Zu erwähnen ist hier eine gute Wirkung gegen Mehltau, Rost, Cochliobolus sativus, Pyrenophora teres an Getreide und gegen Pyricularia oryzae an Reis.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder - schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte ali phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetate, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,1 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die obigen Angaben über die Formulierung und Anwendung der Wirkstoffe gelten nicht nur für die erfindungsgemäßen Bisazolylethercarbinole der allgemeinen Formel (I), sondern auch sinngemäß für die Zwischenprodukte der allgemeinen Formel - (II) (die Azolyl-phenoxy-methyl-oxirane).

Herstellungsbeispiele

Zwecks besserer Übersicht werden die erfindungsgemäßen Bisazolylethercarbinole der allgemeinen Formel (I) mit der Vorzahl I, die Zwischenprodukte (Azolyl-phenoxy-methyl-oxirane) der allgemeinen Formel (II) mit der Vorzahl II bezeichnet.

Beispiel II-1

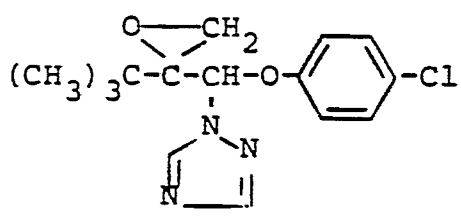

24,4 g (0,11 Mol) Trimethylsulfoxoniumjodid werden in 160 ml Dimethylsulfoxid suspendiert und bei 20°C unter Rühren portionsweise mit 2,8 g (0,1 Mol) 80 %igem Natriumhydrid versetzt. Nach 30 Minuten fügt man 29,3 g (0,1 Mol) 1-(4-Chlorphenoxy)-1-(1,2,4-triazol-1-yl)-3,3-dimethyl-butan-2-on zu, erwärmt und hält das Reaktionsgemisch während 1 Stunde auf 60°C und weiteren 5 Stunden auf 80 bis 90°C. Anschließend gießt man in Wasser, extrahiert mit Methylenchlorid, wäscht die organische Phase mit Wasser und engt im Vakuum ein. Durch Filtrieren des Rohproduktes über eine kurze Kieselgelsäule erhält man das reine 2-[(4-Chlorphenoxy)-(1,2,4-triazol-1-yl)-methyl]-2-(tert.-butyl)-oxiran vom Fp. 89-90°C. Die Ausbeute beträgt 19,7 g (das sind 64 % der Theorie).

Beispiel I-1

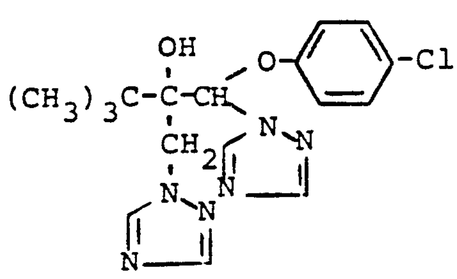

Zur Lösung von 1,5 g (0,065 Mol) Natrium in 150 ml n-Butanol und 10,2 g (0,15 Mol) 1,2,4-Triazol gibt man 0,1 Mol rohes 2-[(4-Chlorphenoxy)-(1,2,4-triazol-1-yl)-methyl]-2-(tert.-butyl)-oxiran (siehe Beispiel II-1) und kocht während 4 Stunden unter Rückfluß. Anschließend wird die Lösung in Vakuum eingeengt, der Rückstand in Methylenchlorid/Wasser aufgenommen, die organische Phase mehrmals mit Wasser gewaschen und getrocknet. Nach dem Abziehen des Lösungsmittels wird das zurückbleibende Öl an Kieselgel chromatographiert (Laufmittel Chloroform/Essigester 9:1). Man erhält 16,8 g (das sind 44,6 % der Theorie) 1-(4-Chlorphenoxy)-1,3-bis-(1,2,4-triazol-1-yl)-2-(tert.-butyl)-propan-2-ol vom Fp. 92-95°C.

Beispiel I-2

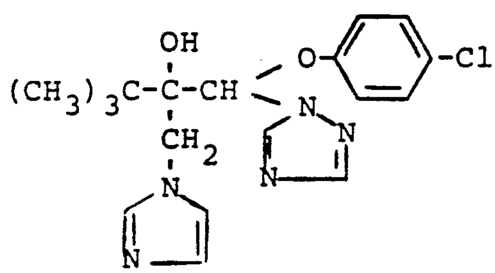

Zur Herstellung der bezeichneten Verbindung verfährt man gemäß Beispiel I-1, jedoch setzt man Imidazol (anstelle von Triazol) ein. Die Aufarbeitung erfolgt in üblicher Weise. Man erhält ein hellgelbes Harz mit dem Brechungsindex $n_D^{20}$ = 1,5373.

Beispiel II-2

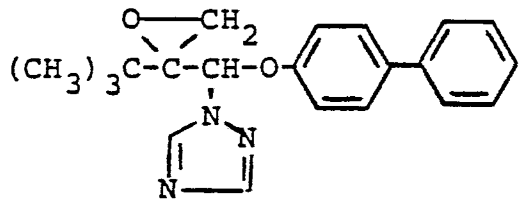

Die Herstellung der bezeichneten Verbindung erfolgt gemäß Beispiel II-1, jedoch setzt man 1-(4-Phenyl-phenoxy)-1-(1,2,4-triazol-1-yl)-3,3-dimethylbutan-2-on (anstelle der entsprechenden 1-(4-Chlorphenoxy)-Verbindung) ein.

Die Aufarbeitung erfolgt in üblicher Weise. Man erhält ein hellgelbes Harz.

Beispiel I-3

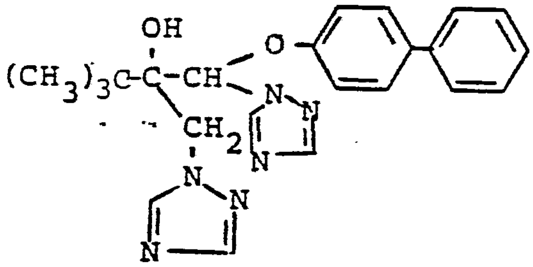

Zur Herstellung der bezeichneten Verbindung verfährt man gemäß Beispiel I-1, jedoch setzt man als Ausgangsprodukte die Verbindung gemäß Beispiel II-2 und 1,2,4-Triazol ein.

Die Aufarbeitung erfolgt in üblicher Weise. Man erhält ein hellgelbes Harz vom Brechungsindex $n_D^{20}$ = 1,5672.

Beispiel I-4

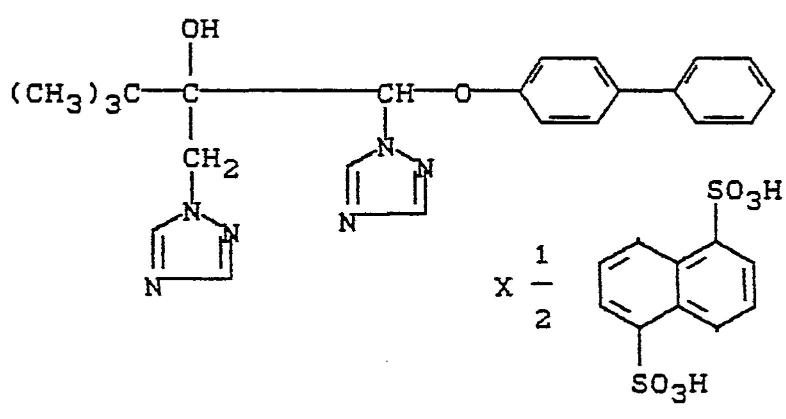

Bei der Behandlung des im Beispiel (I-3) beschriebenen Produktes mit Naphthalin-1,5-disulfonsäure in Aceton erhält man das oben angegebene Säure-Additions-Salz in Form einer kristallinen Verbindung vom Schmelzpunkt 198°C.

Beispiel II-3

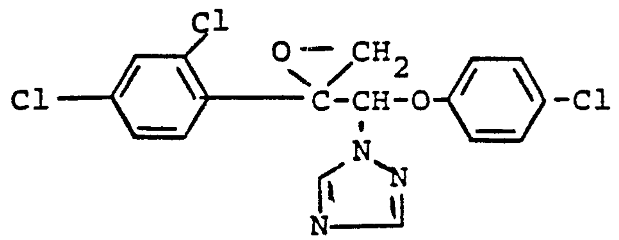

Die Herstellung der bezeichneten Verbindung erfolgt gemäß Beispiel II-1, jedoch setzt man als Ausgangsprodukt 1-(4-Chlorphenoxy)-1-(1,2,4-triazol-1-yl)-2-(2,4-dichlorphenyl)-ethan-2-on ein. Man erhält nach üblicher Aufarbeitung ein hellgelbes Harz.

Beispiel I-5

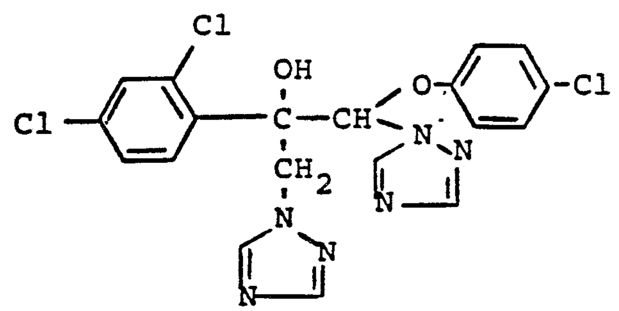

Zur Herstellung der bezeichneten Verbindung verfährt man gemäß Beispiel I-1, jedoch setzt man als Ausgangsprodukte die Verbindung gemäß Beispiel II-3 und 1,2,4-Triazol ein.

Die Aufarbeitung erfolgt in üblicher Weise. Die Verbindung besitzt den Fp. 220°C.

Verwendungsbeispiele

In den nachfolgenden Verwendungsbeispielen wurden als Vergleichssubstanzen eingesetzt:

(A)

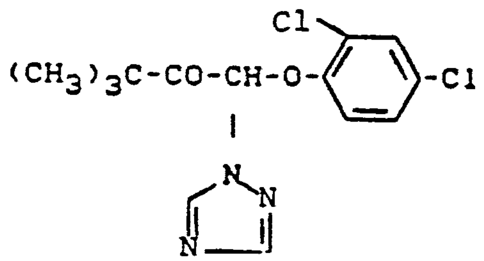

(B)

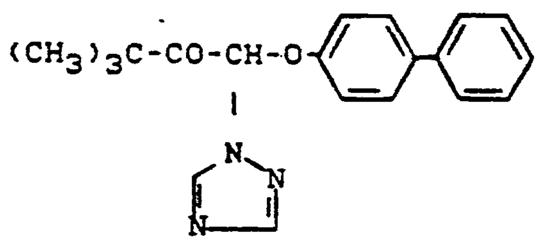

Beispiel A

Leptosphaeria nodorum-Test (Weizen) / protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigen zum Beispiel die erfindungsgemäßen Verbindungen (I-2) und (II-2) eine bessere Wirksamkeit als die Vergleichssubstanz - (A).

Beispiel B

Sphaerotheca-Test (Gurke) / protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen zum Beispiel die erfindungsgemäßen Substanzen (I-1), (I-3), (I-5) und (II-3) eine bessere Wirksamkeit als die Vergleichssubstanzen (A) und (B).

## Ansprüche

1. Bisazolylethercarbinole der allgemeinen Formel - (I)

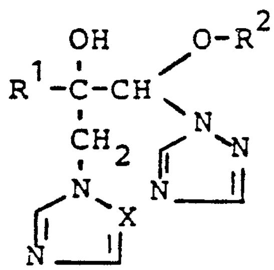

(I)

in welcher

$R^1$ für gegebenenfalls substituiertes Alkyl, Cycloalkyl, für gegebenenfalls substituiertes Aryl und für einen gegebenenfalls substituierten 5-oder 6-gliedrigen Heterocyclus steht,

$R^2$ für gegebenenfalls substituiertes Aryl und für einen gegebenenfalls substituierten 5-oder 6-gliedrigen Heterocyclus steht, und

X für Stickstoff oder die CH-Gruppe steht,

und deren Säureadditionssalze und Metallsalzkomplexe.

2. Verbindungen der allgemeinen Formel (I) in Anspruch 1, in denen

$R^1$ für Alkyl mit 1 bis 6 Kohlenstoffatomen steht, welches substituiert sein kann durch Halogen, Methoxy, Methylthio und Cyano, für Cycloalkyl mit 5 bis 6 Kohlenstoffatomen und weiterhin für Phenyl steht, wobei letzteres gegebenenfalls substituiert sein kann durch Halogen, Alkyl mit 1 bis 4 C-Atomen, Methoxy, Methylthio, Trifluormethyl, Trifluormethoxy und Nitro, und schließlich für einen 5- oder 6-gliedrigen Heterocyclus mit 1 oder 2 Stickstoffatomen und/oder einem Sauerstoff-oder Schwefelatom steht, der substituiert sein kann durch Halogen, Methyl, Methoxy und Methylthio,

$R^2$ für Phenyl steht, welches gegebenenfalls durch die bei $R^1$ genannten Phenylsubstituenten substituiert sein kann, und schließlich für einen 5-oder 6-gliedrigen Heterocyclus mit 1 oder 2 Stickstoffatomen und/oder einem Sauerstoff-oder Schwefelatom steht, der substituiert sein kann durch Halogen, Methyl, Methoxy und Methylthio, und

X für Stickstoff oder die CH-Gruppe steht.

3. Verfahren zur Herstellung von Bisazolylethercarbinolen der allgemeinen Formel (I)

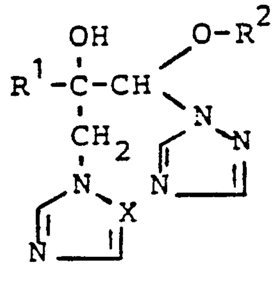

(I)

in welcher

$R^1$ für gegebenenfalls substituiertes Alkyl, Cycloalkyl, für gegebenenfalls substituiertes Aryl und für einen gegebenenfalls substituierten 5-oder 6-gliedrigen Heterocyclus steht,

$R^2$ für gegebenenfalls substituiertes Aryl und für einen gegebenenfalls substituierten 5-oder 6-gliedrigen Heterocyclus steht, und

X für Stickstoff oder die CH-Gruppe steht,

sowie von deren Säureadditions-Salzen und Metallsalz-Komplexen,

dadurch gekennzeichnet, daß man Azolyl-phenoxy-methyl-oxirane der allgemeinen Formel (II)

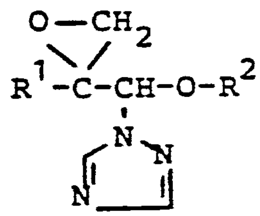

(II)

in welcher

in welcher

$R^1$ und $R^2$ die oben angegebene Bedeutung besitzen,

mit einem Azol der Formel (III)

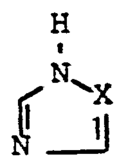

(III)

in welcher

X die oben angegebene Bedeutung besitzt,

gegebenenfalls in Gegenwart eines Lösungsmittels umsetzt, und gegebenenfalls noch an die erhaltenen Verbindungen der Formel (I) anschließend eine Säure oder ein Metallsalz addiert.

4. Fungizide Mittel, gekennzeichnet durch einen Gehalt an mindestens einem Bisazolylethercarbinol der Formel (I) bzw. an einem Säureadditions-Salz oder Metallsalz-Komplex einer Verbindung der Formel (I).

5. Verfahren zur Bekämpfung von Pilzen, dadurch gekennzeichnet, daß man Bisazolylethercarbinole der Formel (I) bzw. deren Säureadditions-Salze oder Metallsalz-Komplexe auf Pilze oder ihren Lebensraum einwirken läßt.

6. Verwendung von Bisazolylethercarbinolen der Formel (I) oder von deren Säureadditions-Salzen oder Metallsalz-Komplexen sur Bekämpfung von Pilzen.

7. Verfahren zur Herstellung von fungiziden Mitteln, dadurch gekennzeichnet, daß man Bisazolylethercarbinole der Formel (I) bzw. deren Säureadditions-Salze oder Metallsalz-Komplexe mit Streckmitteln und/oder oberflächenaktiven Mitteln vermischt.

8. Azolyl-phenoxy-methyl-oxirane der allgemeinen Formel (II)

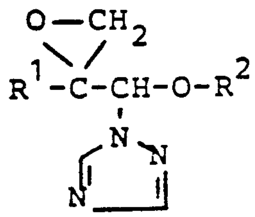

(II)

in welcher

$R^1$ für gegebenenfalls substituiertes Alkyl, Cycloalkyl, für gegebenenfalls substituiertes Aryl und für einen gegebenenfalls substituierten 5-oder 6-gliedrigen Heterocyclus steht, und

$R^2$ für gegebenenfalls substituiertes Aryl und für einen gegebenenfalls substituierten 5-oder 6-gliedrigen Heterocyclus steht.

9. Verfahren zur Herstellung von Azolyl-phenoxy-methyl-oxiranen der allgemeinen Formel (II)

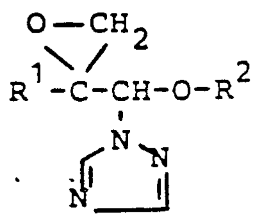

(II)

in welcher

$R^1$ für gegebenenfalls substituiertes Alkyl, Cycloalkyl, für gegebenenfalls substituiertes Aryl und für einen gegebenenfalls substituierten 5-oder 6-gliedrigen Heterocyclus steht, und

$R^2$ für gegebenenfalls substituiertes Aryl und für einen gegebenenfalls substituierten 5-oder 6-gliedrigen Heterocyclus steht,

dadurch gekennzeichnet, daß man Azolyl-phenoxy-keto-Verbindungen der allgemeinen Formel (IV)

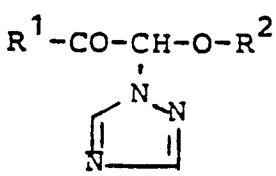

(IV)

in welcher

$R^1$ und $R^2$ die oben angegebenen Bedeutungen besitzen,

mit Trimethylsulfoxoniumjodid in einem polaren Lösungsmittel in Gegenwart einer starken Base zur Reaktion bringt.

10. Verwendung von Azolyl-phenoxy-methyl-oxiranen der allgemeinen Formel (II) zur Bekämpfung von Pilzen.

Europäisches Patentamt

# EUROPÄISCHER RECHERCHENBERICHT

Nummer der Anmeldung

EP 86104719.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 926 280 (BASF)<br>* Ansprüche 1,4 *<br>-- | 1,4 | C 07 D 249/08<br>C 07 D 403/06<br>C 07 D 405/06<br>A 01 N 43/653 |
| A | DE - A1 - 2 918 801 (BASF)<br>* Ansprüche 1,4 *<br>-- | 1,4 | |
| A | DE - A1 - 2 324 010 (BAYER)<br>* Formel I; Ansprüche 3-6 *<br>-- | 1,4-7 | |
| A | DE - A1 - 3 150 204 (BASF)<br>* Anspruch 1 *<br>---- | 1,4 | |
| | | | RECHERCHIERTE SACHGEBIETE (Int. Cl.4) |
| | | | C 07 D 249/00<br>C 07 D 403/00<br>C 07 D 405/00 |

Der vorliegende Recherchenbericht wurde für alle Patentansprüche erstellt.

| Recherchenort | Abschlußdatum der Recherche | Prüfer |
|---|---|---|
| WIEN | 17-07-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument